# EUROPEAN PATENT SPECIFICATION

(11) **EP 2 519 174 B1**
(45) Date of publication and mention of the grant of the patent: **31.07.2019**
(21) Application number: 10801335.0
(22) Date of filing: 21.12.2010
(51) Int. Cl.: A61B 18/04, A61M 1/00, A61M 3/02

(54) **COLLAPSIBLE IRRIGATION CHANNEL FOR PROCEDURES INVOLVING FLUID CIRCULATION**
ZUSAMMENKLAPPBARER BEWÄSSERUNGSKANAL FÜR VERFAHREN MIT FLÜSSIGKEITSZIRKULATION
CANAL D'IRRIGATION TÉLESCOPIQUE POUR DES INTERVENTIONS COMPRENANT LA CIRCULATION DE FLUIDE

(30) Priority: 31.12.2009 US 291515 P
(43) Date of publication of application: 07.11.2012
(73) Proprietor: Boston Scientific Scimed, Inc., Natick, MA 01760-1537 (US)
(72) Inventor: OSTROVSKY, Isaac, Wellesley Massachusetts 02481 (US); SLANDA, Jozef, Milford Massachusetts 01757 (US); SEEHUSEN, Ashley, Palo California 94301 (US)
(74) Representative: Pfenning, Meinig & Partner mbB
(86) International application number: PCT/US2010/061501
(87) International publication number: WO 2011/082026

(56) References cited:
- WO-A2-2009/125387
- US-A1- 2003 023 229
- US-A1- 2009 125 014

## Description

### Priority Claim

The present application claims priority to U.S. Provisional Application Serial No. 61/291,515 entitled "Collapsible Irrigation Channel for Procedures Involving Fluid Circulation" filed on December 31, 2009 to Isaac Ostrovsky, Jozef Slanda and Ashley Seehusen.

### Background

One technique for the treatment of menorrhagia involves the ablation of the uterine lining by supplying heated fluid to the uterus. This heated ablation fluid is supplied via a supply lumen in a device inserted into the uterus via the cervix and withdrawn therefrom via a return lumen in the device. To protect the patient, such systems must closely monitor the volume of fluids flowing into and out of the uterus to ensure that fluids are not being absorbed into the body and/or escaping from the uterus (e.g., through a tear in the uterine wall). This also helps these systems maintain desired levels of efficiency and effectiveness. Such systems generally employ concentric lumens extending through a hysteroscope with a first one of the lumens configured to infuse fluid into the body and a second one of the lumens configured to withdraw the fluids from the body. However, dislodged tissue can be aspirated into the return lumen and circulated through the hysteroscope, jamming one or both of the lumens, restricting flow therethrough and/or interfering with the insertion of instruments through the hysteroscope. Obstruction of the circulation can cause burning or other damage to the uterus and reduce the efficacy of the treatment. Furthermore, the rigid structure of the first and second lumens extending through the hysteroscope prevents the insertion of additional instrumentation therethrough.

WO 2009/125387 A2 discloses a device including an elongated member with a lumen through which a further lumen passes through. The further lumen can be in a collapsed or expanded configuration, if pressure or suction is applied to the proximal end of the lumen. By application of pressure to the inside of the lumen, the expanded or collapsed configuration can be achieved.

US 2009/125014 A1 discloses a thermal ablation system including a device for providing fluid to an organ within a living body comprising an elongate member with a lumen running therethrough. Neither of these documents discloses a portion of a wall configured such that it is maintained in the expanded configuration when a differential between a force directed radially outward against the portion of the wall and a force directed radially inward thereagainst is greater than a positive threshold level.

### Summary of the Invention

The present disclosure is directed to a device for providing fluid to an organ within a living body comprising an elongate member including a distal portion configured for insertion to a target location within a living body. The elongate member includes a withdrawal lumen extending from an outlet in a proximal end thereof to an inlet at a distal end of the elongate member, the inlet opening into a target organ when the distal portion of the elongate member is in the target location. The elongate member further includes an infusion lumen extending from an inlet in the proximal end thereof to an outlet which, when the distal portion of the elongate member is in the target location, opens into the target organ. A first portion of a wall separating the withdrawal lumen from the infusion lumen is configured to move between an expanded configuration and a collapsed configuration in which the cross-sectional area of a first portion of the infusion lumen adjacent to the first portion of the wall is reduced as compared to the expanded configuration so that a cross-sectional area of a first portion of the withdrawal lumen adjacent to the first portion of the wall is greater when the first portion of the wall is in the collapsed configuration than when the first portion of the wall is in the expanded configuration. The scope of the invention is defined by the appended claims.

### Brief Description of the Drawings

The accompanying drawings, which are incorporated in and constitute a part of this specification, illustrate several embodiments of the invention and, together with the description, serve to explain the principles of the invention. In the drawings:
Fig. 1 shows a perspective partial zoom view of a system according to a first exemplary embodiment of the present invention in a first operative configuration; and
Fig. 2 shows a perspective view of the system of Fig. 1 in a second operative configuration.

### Detailed Description

The present invention may be further understood with reference to the following description. The present invention relates to a system for regulating ablative fluid flow into a living body to maintain a desired fluid flow into and out of the body (e.g., by preventing tissue dislodged from an inner surface of a hollow organ from jamming flow paths). In particular, the present invention relates to a collapsible infusion channel which is capable of changing its shape and/or position within a hydrothermal ablation ("HTA") device under forces applied thereto by dislodged tissue, fluid flow, instruments inserted through the device, etc. The exemplary system and method of the present invention are described with respect to devices for ablating the endometrium. However, those skilled in the art will understand that the present invention, and/or components thereof, may be utilized in conjunction with devices for prostate treatment (microwave or cyroablation), irrigation systems or other devices for procedures which infuse fluids to the body.

HTA systems generally utilize one or more resistive heating elements to warm a circulating fluid (e.g., saline) to a predetermined, substantially constant temperature. For example, such a thermal ablation system may heat the ablation fluid within a disposable cassette portion removably coupled to a reusable console. The heated ablation fluid may then be circulated through a hollow organ to ablate the lining thereof and subsequently returned to the system either for recirculation or disposal. One such system is described in a U.S. Provisional Patent Application entitled "Thermal Ablation System", naming as inventors Robert J. Bouthillier, Michael P. Fusaro, Joseph M. Gordon, Stephen S. Keaney, Brian MacLean, Andrew W. Marsella, David Robson and Boris Shapeton filed on November 14, 2007 and assigned Serial No. 60/987,913. The entire disclosure of this application is hereby expressly incorporated by reference herein. The fluid follows a path passing a heating element which elevates the fluid temperature to the desired level. The fluid is then circulated through the hollow organ after which it is filtered and returned to the heating element for recirculation to the organ in a closed loop. A disruption of flow may interrupt circulation of the fluid, thus potentially causing a buildup of fluid in the body and/or overheating the fluid by extending the time during which fluid remains in a fluid reservoir exposed to the heater.

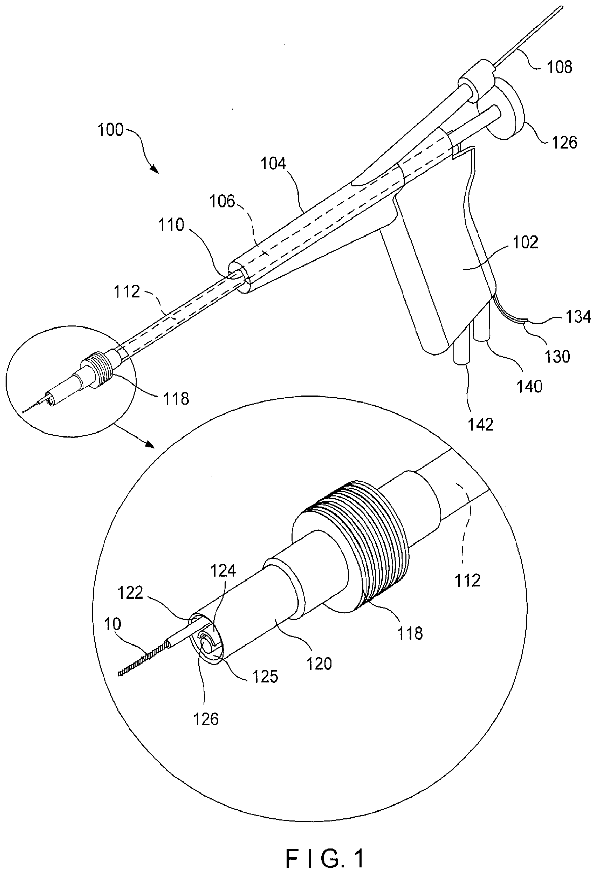

Figs. 1 - 2 depict an ablation device 100 according to an exemplary embodiment of the present invention. The device 100 is an HTA ablation probe comprising a handle 102 and an elongated cannula 104 extending distally therefrom. The cannula 104 comprises a lumen 106 extending therethrough from a proximal opening (not shown) to a distal opening 110 permitting engagement with a reduced diameter cannula 112 extending distally therefrom. Specifically, the cannula 112 may be attached to a distal end of the cannula 104 or, in another embodiment, may extend through the cannula 104. The cannula 112, which is configured for insertion into a target organ within a living body (e.g., via a body lumen accessed via a naturally occurring body orifice) comprises a seal 118 at a distal end thereof which is configured to extend around and seal an opening of the uterus or another body cavity to prevent heated fluids injected thereinto from leaking out of the uterus. The seal 118 is configured so that an outer wall thereof engages the inner diameter of the cervix to prevent fluid leakage from the uterus. Those skilled in the art will understand that the accordion shape and flexibility of the fins of the seal 118 increase a frictional engagement between the device 100 and the wall of the cervix to ensure secure engagement therewith. Furthermore, as the device 100 is inserted into the cervix, the fins of the seal 118 flex proximally, facilitating the insertion. A user may insert the device 100 into the uterus to a depth sufficient to pass all of the fins beyond the distal end of the cervix and then withdraw the device 100 proximally to seat the fins against the cervical os to fully seal the cervix and prevent heated fluid from passing thereinto and injuring non-targeted tissue. The seal 118 is attached to a distal section 120 having different material properties than the cannula 112. Specifically, the distal section 120 may be more or less flexible than the cannula 112 and/or may have a different diameter than the cannula 112. The distal section 120 comprises a lumen 122 extending therethrough. A proximal end of the lumen 122 is aligned with and open to a distal end of the lumen 106 of the cannula 112. Furthermore, it is noted that the distal section 120 may be formed of any desired length without deviating from the scope of the present invention.

A conduit 124 extending through the cannula 112 and the distal section 120 has a length at least as long as the combined length of the cannula 112 and the distal section 120. The conduit 124 is formed with a collapsible, thin wall having a stiffness selected so that, when subject to the forces to which the conduit 124 will be exposed in use (e.g., the bending forces to which such flexible medical instruments are subject intralumenally as well as fluid pressures within and external to the conduit 124), at least a portion of the conduit 124 collapses, moves and/or flexes to adapt to different conditions such as a blockage (e.g., due to detached tissue) or the need to insert an instrument through the device 100 and into the uterus. That is, when a pressure outside the conduit 124 exceeds the pressure therewithin by a differential greater than a threshold level at any point along the length of the conduit 124, that portion of the conduit 124 collapses to a reduced area configuration in which a wall of the conduit 124 is moved radially inward reducing a cross-sectional area thereof. When the differential between the pressure external to the conduit 124 and the pressure therewithin is less than the threshold level, the wall of the conduit 124 is moved radially outward to an expanded configuration in which a cross-sectional area thereof is greater than it is in the collapsed configuration. The wall of the conduit 124 is configured so that a certain level of internal pressure (e.g., 133.3 Pa - 400 Pa (1-3 mm Hg)) is required to maintain the conduit 124 in the expanded configuration. For example, a force applied by an endoscope or other medical instrument inserted through the cannula 112 may collapse one or more parts of the conduit 124 depending, for example, on the curvature of the cannula 112 along its length, inward pressure due to contact with body structures, etc. Those skilled in the art will recognize that the conduit 124 may be used as an inlet passage to supply fluids to the target body organ. Thus, if for any reason it is desired to move the conduit 124 to the collapsed configuration, fluid supply to this lumen may be reduced or halted to reduce the pressure therein. For example, if dislodged tissue from the body becomes clogged in a portion of the lumen 122, the fluid supply through the conduit 124 may be reduced or halted to cause the conduit to collapse and increase the dimensions of a clearance space 125 located therearound. The increased clearance space 125 permits the dislodged tissue to move through the lumen 112 and out of a withdrawal port 140 located on the handle 102.

In another embodiment, a force applied to an outer wall of the conduit 124 by the dislodged tissue flowing through the cannula 112 may cause at least a partial collapse of the conduit 124, temporarily expanding that portion of the cannula 112 and permitting the dislodged tissue to move therepast to the withdrawal port 140 on the handle 102, as will be described in greater detail later on. In yet another embodiment, negative pressure may be applied to the cannula 112 externally of the conduit 124 to cause the conduit 124 to collapse, especially in the absence of a positive fluid flow through the conduit 124. It is noted that the collapse of the conduit 124 may be facilitated by any other means without deviating from the present invention as claimed. The conduit 124 may also be configured with behavioral properties that vary along its length. Specifically, predetermined sections of the conduit 124 may be configured to be more easily collapsed to permit inflation of these areas when an excessive pressure is reached within the conduit. Thus, a physician or other user may collapse the conduit 124 to prevent a further buildup of pressure or bursting of the conduit 124.

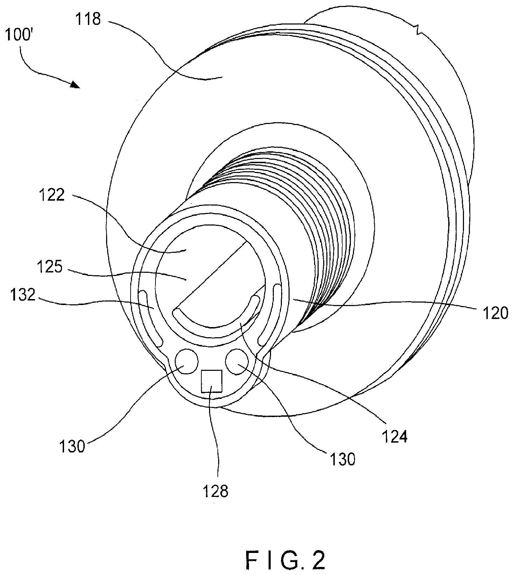

The conduit 124 is formed of a layer of Teflon ®, polyethylene or any other substantially resilient and/or elastic polymer material having a thickness of approximately 0.0127 mm. - 0.254 mm. An outer diameter of the conduit 124 may be selected based on the inner diameter of the cannula 112 and the distal section 120 - i.e., to provide any desired clearance space 125 between the outer surface of the conduit 124 and an inner surface of the cannula 112. In one embodiment, the cannula 112 may have an outer diameter of approximately 6 mm. Although conventional hydrothermal ablation devices have generally included a rigid lumen concentrically placed within an outer channel, the conduit 124 of the present invention is configured to warp and change a diameter and cross-sectional shape thereof to accommodate changes in fluid flow through the cannula 112 and/or to facilitate the insertion of instruments therethrough. Specifically, the conduit 124 may warp to change a cross-sectional shape thereof as an instrument is inserted therethrough or as a fluid clearance space 125 within the cannula 112 changes. The conduit 124, which provides inlet flow of fluid to the uterus, is formed of a material having a flexibility sufficient to permit the conduit 124 to fold in on itself to a collapsed configuration when not pressurized. In this collapsed configuration, the conduit 124 provides a clearance 125 of approximately 3 mm. for the insertion of an instrument through the cannula 112 and the distal section 120. The material of the conduit 124 is preferably be selected so that a pressure provided by a fluid flowing therethrough is sufficient (e.g., 200-350 ml/min when uterine pressure is 65 mmHg.) to maintain the conduit 124 in a non-collapsed, substantially cylindrical configuration. The thin walls of the conduit 124 permit the conduit 124 to assume a cross-sectional shape matching a cross-sectional shape of its boundaries, wherein the possible shapes include, but are not limited to elliptical and oblong. If no outer boundaries are present, the cross-sectional shape of the conduit 124 may be substantially cylindrical. The conduit 124 is also sized so that when an endoscope eyepiece 126 is inserted through the device 100, the clearance space 125 is provided with predetermined dimensions to permit fluid to be withdrawn from the body, as those skilled in the art will understand. The endoscope 126 may be inserted into the lumen 106 and through the lumen 122 at the same time that an instrument 10 is inserted through the instrument port 108 located on the handle 102. When the conduit 124 is pressurized, the clearance space 125 provided for the endoscope may be reduced to approximately 2 mm. Thus, when there is a need to insert another instrument (e.g., a sterilization coil, etc.) through the lumens 106, 122, the conduit 124 may be moved to the collapsed configuration as shown in Fig. 2 or may assume the partially warped configuration shown in Fig. 1.

The conduit 124 is configured to warp an outer diameter thereof to a plurality of cross-sectional shapes without affecting a flow volume and flow rate of fluid passing therethrough. In the non-pressurized configuration, the conduit 124 assumes an oblong shape having a substantially arced cross-sectional shape so that the conduit 124 may be seated against a side wall of the lumen 122 to maximize the size of the clearance space 125. It is noted however, that the material of the conduit 124 may be pre-formed to hold any shape within the lumens 106, 122 as would be understood by those skilled in the art. In one example, pressure applied to the walls of the conduit 124 by the instrument 10 causes the conduit 124 to assume a different shape to accommodate movement of the instrument 10 within the lumens 106, 122. Accordingly, depending on the size of the instrument to be inserted through the lumen 122, a flow through the conduit 124 may remain unchanged, be reduced to a lower flow rate to permit a partial collapse of outer walls thereof or may be terminated, as shown in Fig. 1, wherein the exemplary flow rate may be selected to maintain the patient organ or cavity in an open configuration for visualization while still preventing overpressurization thereof. The lumens 122, 106 are open to the fluid withdrawal port 140 located on the handle while the conduit 124 is open to a fluid supply port 142 on the handle to permit the introduction of heated fluid to a target organ in the living body.

Fig. 2 depicts another embodiment of the present invention, wherein the endoscope 126 may be removed to further increase the size of the clearance space 125 within the distal section 120 and the cannula 112. Specifically, in this embodiment, the distal face 132 of this apparatus includes a camera 128 and light fibers 130 which from this distal face 132 to the proximal end of the device at which they may be coupled to a source of light to illuminate a field of view of the camera 128 thereby eliminating the need for an endoscope 126 as described in the previous embodiment. Specifically, the light fibers 130 may extend within an outer wall of the device 100' to a proximal end thereof to a connection with a light source. The camera 128 transmits an image signal via a signal cable 134 extending through the outer wall of the device 100' on the handle 102 for connection to a processor or other computing device which may be housed within the device or external thereto. In this configuration, a greater clearance space 125 is provided in the lumen 122 for the insertion of other instruments therethrough. Thus, if desired, the conduit 124 may made larger than the conduit 124 of Fig. 1 or, in another embodiment, the distal section 120 and cannula 112 may be made smaller to, for example, facilitate insertion of the device to locations inaccessible to larger instruments. Furthermore, since the light fibers 130 are substantially flexible, the device 100' permits the insertion of nonplanar tools therethrough such as, for example, a sterilization coil delivery tool including a probe tip which may be articulated to a predetermined orientation prior to advancing the delivery tool through the device 100'.

It is to be understood that these embodiments have been described in an exemplary manner and are not intended to limit the scope of the invention which is intended to cover all modifications and variations of this invention that come within the scope of the appended claims and their equivalents.

## Claims

1. A device (100) for providing fluid to an organ within a living body, comprising:
an elongate member including a distal portion (120) configured for insertion to a target location within a living body, the elongate member including a withdrawal lumen extending therewithin from an outlet in a proximal end of the elongate member to an inlet at a distal end of the elongate member, the inlet opening into a target organ when the distal portion of the elongate member is in the target location, the elongate member further including an infusion lumen, the infusion lumen extending from an inlet in the proximal end of the elongate member to an outlet which, when the distal portion of the elongate member is in the target location, opens into the target organ,
wherein the elongate member includes a wall separating the withdrawal lumen from the infusion lumen, a first portion of the wall being configured to move between an expanded configuration and a collapsed configuration in which a cross-sectional area of the first portion of the infusion lumen adjacent to the first portion of the wall is reduced as compared to the expanded configuration so that a cross-sectional area of a first portion of the withdrawal lumen adjacent to the first portion of the wall is greater when the first portion of the wall is in the collapsed configuration than when the first portion of the wall is in the expanded configuration,
wherein the first portion of the wall is configured such that it is maintained in the expanded configuration when a differential between a force directed radially outward against the first portion of the wall and a force directed radially inward thereagainst is greater than a positive threshold level.

2. The device of claim 1, wherein the wall includes a plurality of portions movable between expanded and collapsed configurations spaced along an axial length of the wall.

3. The device of claim 2, wherein the threshold level is substantially the same for all of the plurality of portions of the wall.

4. The device of claim 1, wherein the threshold level is selected so that a level of fluid pressure associated with a desired fluid flow to be provided to the target organ via the infusion lumen is sufficient to maintain the first portion of the wall in the expanded configuration when a fluid pressure within the withdrawal lumen is maintained within a target range of fluid pressures.

5. The device of claim 4, wherein the first portion of the wall is designed to move to the collapsed configuration when the supply of fluid to the infusion lumen is suspended.

6. The device of claim 1, wherein the first portion of the wall is formed of a polymer.

7. The device of claim 6, wherein the first portion of the wall is formed of one of Teflon® and polyethylene.

8. The device of claim 1, wherein, when in the expanded configuration, the infusion lumen is substantially circular in cross-section.

9. The device of claim 1, wherein, when in the expanded configuration, the infusion lumen has an oblong cross-section.

10. The device of claim 1, further comprising:
a seal (118) at a distal end of the elongate member configured to rest against tissue of the target organ to prevent leakage of fluids around the elongate member out of the target organ when the distal portion of the elongate member is in the target location.

11. The device of claim 10, wherein a distal portion of the withdrawal lumen has a greater elasticity than a proximal portion thereof.

12. The device of claim 1, wherein the elongate member is sized and shaped for insertion into a uterus via a cervix for hydrothermal ablation of the lining of the uterus.

13. The device of claim 1, further comprising:
a camera (128) and a series of light fibers (130) mounted on a distal end of the elongate member.

14. A system for providing fluid to an organ including device of claim 1, wherein the withdrawal lumen is coupled to a withdrawal port of a handle disposed at a proximal end of an insertion device configured for insertion into the living body, the withdrawal port configured to connect to a withdrawal reservoir, and wherein the infusion lumen is coupled to an infusion port of the handle, the infusion port configured for attachment to an infusion reservoir.

## Patentansprüche

1. Vorrichtung (100) zum Zuführen von Fluid zu einem Organ innerhalb eines lebenden Körpers, welche aufweist:
ein längliches Teil enthaltend einen distalen Bereich (120), das für das Einführen zu einem Zielort innerhalb eines lebenden Körpers konfiguriert ist, welches längliche Teil ein Zurückziehungslumen enthält, das sich darin von einem Auslass in einem proximalen Ende des länglichen Teils zu einem Einlass an einem distalen Ende des länglichen Teils erstreckt, wobei sich der Einlass in ein Zielorgan öffnet, wenn sich der distale Bereichs des länglichen Teils in dem Zielort befindet, welches längliche Teil weiterhin ein Infusionslumen enthält, das sich von einem Einlass in dem proximalen Ende des länglichen Teils zu einem Auslass, der sich, wenn der distale Bereich des länglichen Teils sich in dem Zielort befindet, in das Zielorgan öffnet, erstreckt,
wobei das längliche Teil eine Wand enthält, die das Zurückziehungslumen von dem Infusionslumen trennt, wobei ein erster Bereich der Wand konfiguriert ist, sich zwischen einer expandierten Konfiguration und einer zusammengelegten Konfiguration, in der eine Querschnittsfläche des ersten Bereichs des Infusionslumens benachbart dem ersten Bereich der Wand verkleinert ist im Vergleich zu der expandierten Konfiguration, zu bewegen, so dass eine Querschnittsfläche eines ersten Bereichs des Zurückziehungslumens benachbart dem ersten Bereich der Wand größer ist, wenn der erste Bereich der Wand in der zusammengelegten Konfiguration ist, als wenn der erste Bereich der Wand in der expandierten Konfiguration ist,
wobei der erste Bereich der Wand derart konfiguriert ist, dass er in der expandierten Konfiguration gehalten wird, wenn eine Differenz zwischen einer Kraft, die radial nach auswärts gegen den ersten Bereich der Wand gerichtet ist, und einer Kraft, die radial nach innen dagegen gerichtet ist, größer als ein positiver Schwellenwertpegel ist.

2. Vorrichtung nach Anspruch 1, bei der die Wand mehrere Bereiche enthält, die bewegbar zwischen expandierten und zusammengelegten Konfigurationen sind und entlang einer axialen Länge der Wand einen gegenseitigen Abstand aufweisen.

3. Vorrichtung nach Anspruch 2, bei der der Schwellenwertpegel im Wesentlichen der gleiche für sämtliche der mehreren Bereiche der Wand ist.

4. Vorrichtung nach Anspruch 1, bei der der Schwellenwertpegel so ausgewählt ist, dass ein Pegel von Fluiddruck, der mit einer gewünschten Fluidströmung, die über das Infusionslumen zu dem Zielorgan bereitzustellen ist, assoziiert ist, ausreichend ist, um den ersten Bereich der Wand in der expandierten Konfiguration zu halten, wenn ein Fluiddruck innerhalb des Zurückziehungslumens innerhalb eines Zielbereichs von Fluiddrücken gehalten wird.

5. Vorrichtung nach Anspruch 4, bei der der erste Bereich der Wand so gestaltet ist, dass er sich in die zusammengelegte Konfiguration bewegt, wenn die Zuführung von Fluid zu dem Infusionslumen ausgesetzt wird.

6. Vorrichtung nach Anspruch 1, bei der der erste Bereich der Wand aus einem Polymer gebildet ist.

7. Vorrichtung nach Anspruch 6, bei der der erste Bereich der Wand aus einem von Teflon® und Polyethylen gebildet ist.

8. Vorrichtung nach Anspruch 1, bei der das Infusionslumen, wenn es in der expandierten Konfiguration ist, im Querschnitt im Wesentlichen kreisförmig ist.

9. Vorrichtung nach Anspruch 1, bei der das Infusionslumen, wenn es in der expandierten Konfiguration ist, einen länglichen Querschnitt hat.

10. Vorrichtung nach Anspruch 1, welche weiterhin aufweist:
eine Abdichtung (118) an einem distalen Ende des länglichen Teils, die konfiguriert ist, an Gewebe des Zielorgans anzuliegen, um ein Entweichen von Fluiden um das längliche Teil herum aus dem Zielorgan heraus zu verhindern, wenn sich der distale Bereichs des länglichen Teils in dem Zielort befindet.

11. Vorrichtung nach Anspruch 10, bei der ein distaler Bereich des Zurückziehungslumens eine größere Elastizität hat als ein proximaler Bereich hiervon.

12. Vorrichtung nach Anspruch 1, bei der das längliche Teil bemessen und geformt ist für die Einführung in eine Gebärmutter über eine Cervix für hydrothermale Ablation der Auskleidung der Gebärmutter.

13. Vorrichtung nach Anspruch 1, weiterhin aufweisend:
eine Kamera (128) und eine Reihe von Lichtfasern (130), die auf einem distalen Ende des länglichen Teils befestigt sind.

14. System zum Liefern von Fluid zu einem Organ, das die Vorrichtung nach Anspruch 1 enthält, wobei das Zurückziehungslumen mit einem Zurückziehungsport eines Handgriffs, der an einem proximalen Ende einer Einführungsvorrichtung, die für die Einführung in den lebenden Körper konfiguriert ist, angeordnet ist, gekoppelt ist, wobei der Zurückziehungsport konfiguriert ist zum Verbinden mit einem Zurückziehungsbehälter, und wobei das Infusionslumen mit einem Infusionsport des Handgriffs gekoppelt ist, welcher Infusionsport für die Anbringung an einem Infusionsbehälter konfiguriert ist.

## Revendications

1. Dispositif (100) pour alimenter un fluide jusqu'à un organe à l'intérieur du corps d'un être vivant, comprenant :
un élément allongé qui inclut une section distale (120) qui est configurée de manière à ce qu'elle puisse être insérée jusqu'à une localisation cible à l'intérieur du corps d'un être vivant, l'élément allongé incluant une lumière de prélèvement qui s'étend à l'intérieur de lui-même depuis une sortie au niveau d'une extrémité proximale de l'élément allongé jusqu'à une entrée au niveau d'une extrémité distale de l'élément allongé, l'entrée débouchant à l'intérieur d'un organe cible lorsque la section distale de l'élément allongé est au niveau de la localisation cible, l'élément allongé incluant en outre une lumière de perfusion, la lumière de perfusion s'étendant depuis une entrée au niveau de l'extrémité proximale de l'élément allongé jusqu'à une sortie qui, lorsque la section distale de l'élément allongé est au niveau de la localisation cible, débouche à l'intérieur de l'organe cible ; dans lequel :
l'élément allongé inclut une paroi qui sépare la lumière de prélèvement vis-à-vis de la lumière de perfusion, une première section de la paroi étant configurée de manière à ce qu'elle soit déplacée entre une configuration déployée et une configuration repliée dans laquelle une aire en coupe transversale de la première section de la lumière de perfusion qui est adjacente à la première section de la paroi est réduite par comparaison avec la configuration déployée de telle sorte qu'une aire en coupe transversale d'une première section de la lumière de prélèvement qui est adjacente à la première section de la paroi soit plus importante lorsque la première section de la paroi est dans la configuration repliée que lorsque la première section de la paroi est dans la configuration déployée ; dans lequel :
la première section de la paroi est configurée de telle sorte qu'elle soit maintenue dans la configuration déployée lorsqu'un différentiel entre une force qui est dirigée radialement vers l'extérieur contre la première section de la paroi et une force qui est dirigée radialement vers l'intérieur contre cette même première section de la paroi est plus important qu'un niveau de seuil positif.

2. Dispositif selon la revendication 1, dans lequel la paroi inclut une pluralité de sections qui peuvent être déplacées entre des configurations déployée et repliée selon un espacement suivant une longueur axiale de la paroi.

3. Dispositif selon la revendication 2, dans lequel le niveau de seuil est sensiblement le même pour toutes les sections de la pluralité de sections de la paroi.

4. Dispositif selon la revendication 1, dans lequel le niveau de seuil est sélectionné de telle sorte qu'un niveau d'une pression de fluide qui est associée à un écoulement de fluide souhaité qui est destiné à être alimenté jusqu'à l'organe cible via la lumière de perfusion soit suffisant de manière à maintenir la première section de la paroi dans la configuration déployée lorsqu'une pression de fluide à l'intérieur de la lumière de prélèvement est maintenue à l'intérieur d'une plage cible de pressions de fluide.

5. Dispositif selon la revendication 4, dans lequel la première section de la paroi est conçue de manière à ce qu'elle soit déplacée jusqu'à la configuration repliée lorsque l'alimentation en fluide sur la lumière de perfusion est suspendue.

6. Dispositif selon la revendication 1, dans lequel la première section de la paroi est formée en un polymère.

7. Dispositif selon la revendication 6, dans lequel la première section de la paroi est formée soit en Téflon®, soit en polyéthylène.

8. Dispositif selon la revendication 1, dans lequel, lorsqu'elle est selon la configuration déployée, la lumière de perfusion présente une section en coupe transversale sensiblement circulaire.

9. Dispositif selon la revendication 1, dans lequel, lorsqu'elle est selon la configuration déployée, la lumière de perfusion présente une section en coupe transversale sensiblement oblongue.

10. Dispositif selon la revendication 1, comprenant en outre :
une étanchéité (118) au niveau d'une extrémité distale de l'élément allongé qui est configurée de manière à ce qu'elle repose contre un tissu de l'organe cible de manière à ce qu'elle empêche une fuite de fluides autour de l'élément allongé hors de l'organe cible lorsque la section distale de l'élément allongé est au niveau de la localisation cible.

11. Dispositif selon la revendication 10, dans lequel une section distale de la lumière de prélèvement présente une élasticité plus importante que sa section proximale.

12. Dispositif selon la revendication 1, dans lequel l'élément allongé est dimensionné et conformé dans le but de son insertion à l'intérieur d'un utérus via un col de l'utérus pour une ablation hydro-thermique de la muqueuse utérine.

13. Dispositif selon la revendication 1, comprenant en outre :
une caméra (128) et une série de fibres optiques (130) qui sont montées sur une extrémité distale de l'élément allongé.

14. Système pour alimenter un fluide jusqu'à un organe, incluant un dispositif selon la revendication 1, dans lequel la lumière de prélèvement est couplée à un orifice de prélèvement d'une poignée qui est disposée au niveau d'une extrémité proximale d'un dispositif d'insertion qui est configuré de manière à réaliser une insertion à l'intérieur du corps de l'être vivant, l'orifice de prélèvement étant configuré de manière à ce qu'il soit connecté à un réservoir de prélèvement, et dans lequel la lumière de perfusion est couplée à un orifice de perfusion de la poignée, l'orifice de perfusion étant configuré de manière à ce qu'il puisse être fixé sur un réservoir de perfusion.
